# EUROPÄISCHE PATENTSCHRIFT

(11) **EP 1 751 080 B1**
(45) Veröffentlichungstag und Bekanntmachung des Hinweises auf die Patenterteilung: **02.12.2009**
(21) Anmeldenummer: 05701394.8
(22) Anmeldetag: 10.02.2005
(51) Int. Cl.: C07C 29/151, C01B 3/58, C01B 3/52, C01B 3/02, C01B 3/38, C01C 1/04

(54) **KOPRODUKTION VON METHANOL UND AMMONIAK AUS ERDGAS**
COPRODUCTION OF METHANOL AND AMMONIA FROM NATURAL GAS
COPRODUCTION DE METHANOL ET D'AMMONIAC A PARTIR DE GAZ NATUREL

(30) Priorität: 22.03.2004 DE 102004014292
(43) Veröffentlichungstag der Anmeldung: 14.02.2007
(73) Patentinhaber: Lurgi GmbH, 60295 Frankfurt am Main (DE)
(72) Erfinder: DAVEY, William, 60439 Frankfurt am Main (DE); WURZEL, Thomas, 61440 Oberursel (DE)
(74) Vertreter: Meyer-Dulheuer, Karl-Hermann
(86) Internationale Anmeldenummer: PCT/EP2005/001330
(87) Internationale Veröffentlichungsnummer: WO 2005/095313

(56) Entgegenhaltungen:
- EP-A- 0 553 631
- WO-A-03/106393
- DE-A1- 2 904 008
- DE-A1- 3 336 649
- DE-A1- 3 712 008
- US-A1- 2002 098 132

## Beschreibung

Die Erfindung betrifft ein Verfahren zur Koproduktion, das heißt zur gleichzeitigen Erzeugung von Methanol und Ammoniak aus Erdgas in einem einzigen ununterbrochenen Prozessablauf.

Es ist eine weit verbreitete Praxis bei der industriellen Gewinnung von Methanol und Ammoniak, getrennte Prozesse zu entwickeln und aufzubauen, um jeweils eines dieser beiden Produkte zu erzeugen. Aus ökonomischer Sicht vorteilhaft ist es dagegen, sowohl Methanol als auch Ammoniak in einem einzigen Prozessablauf herzustellen, denn dadurch werden die Kosten gegenüber zwei getrennten Prozessabläufen für jedes Produkt erheblich gemindert. Die Kosten werden nicht nur während der Durchführung des Herstellungsverfahrens gesenkt, sondern auch schon durch verringerte Anschaffungen für die erforderlichen Ausrüstungsteile zur Herstellung der Anlage.

Die Integration der Methanolherstellung und der Ammoniakherstellung in einem einzigen Verfahren ist seit langem bekannt, vgl. hierzu beispielsweise die US 3 598 527. Die Synthese von Methanol und von Ammoniak erfolgen dabei hintereinander, und das Reinigungsgas aus der zuerst durchgeführten Synthese wird der nachfolgenden Synthese zugeleitet. Demgegenüber ist aus der DE 696 08 301 T2 ein Verfahren mit zwei separaten Prozessen zur Gewinnung von Methanol und Ammoniak bekannt, wobei der Dampfreformer bei der Gewinnung von Methanol seine Energie aus der Gewinnung von Ammoniak erhält.

Alle diese Verfahren haben zum Nachteil, dass die Synthese von Methanol und die Synthese von Ammoniak nicht gleichzeitig und unabhängig voneinander aus einem einzigen Synthesegasstrom erfolgen.

Aus der DE 102 26 209 A1 ist die Zerlegung eines Synthesegases bekannt, wobei Methanol-Synthesegas, Ammoniak-Synthesegas, Kohlenmonoxid und Kohlendioxid gewonnen werden.

Des Weiteren ist in der DE 37 12 008 A1 ein Verfahren zur gleichzeitigen Erzeugung von Methanol und Kohlenmonoxid aus einem Gemisch leichter Kohlenwasserstoffe beschrieben. Die Methanol und CO Synthese kann mit einem an sich bekannten Verfahren zur Ammoniaksynthese verknüpft werden. Hierbei handelt es sich um eine übliche Kopplung der Ammoniaksynthese an die Methanolsynthese. Nach US 2002/0098132 A1 ist ein Verfahren bekannt, nachdem eine ursprüngliche Anlage zur Methanolsynthese beispielsweise in eine solche zur Wasserstoffsynthese aus Methanol umgewandelt werden kann. Dabei wird ein reines Steam-Reforming-Verfahren für die Reformierung des Erdgases beschrieben, welches besonders für die Methanolsynthese geeignet ist.

Aufgabe der vorliegenden Erfindung ist es, ein Verfahren zur Verfügung zu stellen, mit dem gleichzeitig Methanol und Ammoniak in einem einzigen integrierten Prozess hergestellt werden.

Die Aufgabe wird gelöst durch ein Verfahren mit den Schritten von Anspruch 1.

Vorteilhafte Ausführungsformen sind Gegenstand der Unteransprüche.

Mit dem erfindungsgemäßen Verfahren lassen sich z.B. 5.000 t Methanol pro Tag gewinnen. Die Synthese von Methanol erfordert hierbei eine Synthesegaszusammensetzung mit einer Stöchiometriezahl von 2.05 und einer Kohlendioxidkonzentration im Bereich zwischen 2% und 3%. Die Stöchiometriezahl Sₙ wird nach folgender Formel berechnet:

Sₙ = ([H₂]-[CO₂])/([CO₂]+[CO]).

Hierbei stehen die Größen [H₂], [CO₂] und [CO] für die Molenbrüche von Wasserstoff, Kohlendioxid und Kohlenmonoxid, wie sie jeweils in dem Synthesegas vorliegen.

Darüber hinaus lassen sich mit dem erfindungsgemäßen Verfahren z.B. 4.000 t Ammoniak pro Tag erzeugen. Ein Teil hiervon kann zur Herstellung von Harnstoff verwendet werden. Für die Synthese von Ammoniak benötigt man eine Mischung aus Wasserstoff und Stickstoff im Molverhältnis von 3:1 und mit weniger als 10 ppm an Bestandteilen, in denen Sauerstoff enthalten ist.

Weiterhin können z.B. 6.800 t Harnstoff pro Tag hergestellt werden. Die Synthese von Harnstoff erfordert reinen Ammoniak und Kohlendioxid mit einem Reinheitsgrad von mehr als 98.5%.

Diese Erfordernisse werden erfüllt durch die folgende Vorgehensweise:
Aus Erdgas (Strom 1), Dampf und einer Lufttrennungseinheit G entnommenem Sauerstoff wird in dem Reaktor A bei einem Druck von ungefähr 40 bar Rohsynthesegas erzeugt (Ströme 2 und 3).

Das Rohsynthesegas aus dem Reaktor A wird in einen Strom für die Methanolsynthese (Strom 2) und einen Strom zu dem Kohlenmonoxid-Konversionsreaktor im Reaktor B aufgespalten (Strom 3). Die Aufspaltung erfolgt dabei in einem Verhältnis, dass dem Reaktor B mehr Gas zugeleitet wird als für die Methanolsynthese verwendet wird.

Das gekühlte und kondensierte Gas (Strom 4) wird in dem Verdichter und Absorber C gegebenenfalls komprimiert und einer Kohlendioxid-Absorptionskolonne zugeführt. Das kohlendioxidfreie Gas wird einer Reinigungseinheit D zugeführt (Strom 5), das gewonnene Kohlendioxid wird für die Harnstoffsynthese abgezweigt (Strom 14) und verbleibende Verunreinigungen werden dem Brennstoffsystem zugeführt. Der Rest des Gases wird in einer Feinwaschsektion der Absorptionskolonne behandelt, um eine Kohlendioxidkonzentration von weniger als 10 ppm zu erlangen, und wird anschließend ebenfalls zur Reinigungseinheit D geleitet (Strom 5).

Im Gas zurückgebliebene Verunreinigungen werden in der Reinigungseinheit D mit aus der Lufttrennungseinheit G entnommenem flüssigem Stickstoff (Strom 12) durch Druckwechselabsorption an einem Molekularsieb entfernt. Der dabei entstehende Wasserstoff wird aufgespalten in zwei Ströme. Der eine (Strom 6), wird dem Methanol-Synthesegasstrom (Strom 7) zugeleitet, um das korrekte stöchiometrische Verhältnis von 2.05 herbeizuführen, und der zweite wird mit Stickstoff von der Lufttrennungseinheit G (Strom 12) vermischt, um Ammoniak-Synthesegas (Strom 8) für die Einheit F zu erhalten. Verunreinigungen in Form von Methan, Argon und Kohlenmonoxid werden in das Brennstoffsystem zurückgeführt, um als Brennstoff für die im Prozess verwendeten Öfen zu dienen.

In den Einheiten E und F werden der Methanol-Synthesegasstrom und der Ammoniak-Synthesegasstrom (Ströme 7 und 8) jeweils in Methanol (Strom 9) und Ammoniak (Strom 10) umgewandelt. Das Methanol wird in der Einheit E durch Destillation gereinigt, und in der Einheit F wird reines Ammoniak gewonnen, welches keine weitergehende Reinigung erforderlich macht.

Wie oben bereits erwähnt, wird das Kohlendioxid für die Harnstoffsynthese aus dem Verdichter und Absorber C gewonnen (Strom 14).

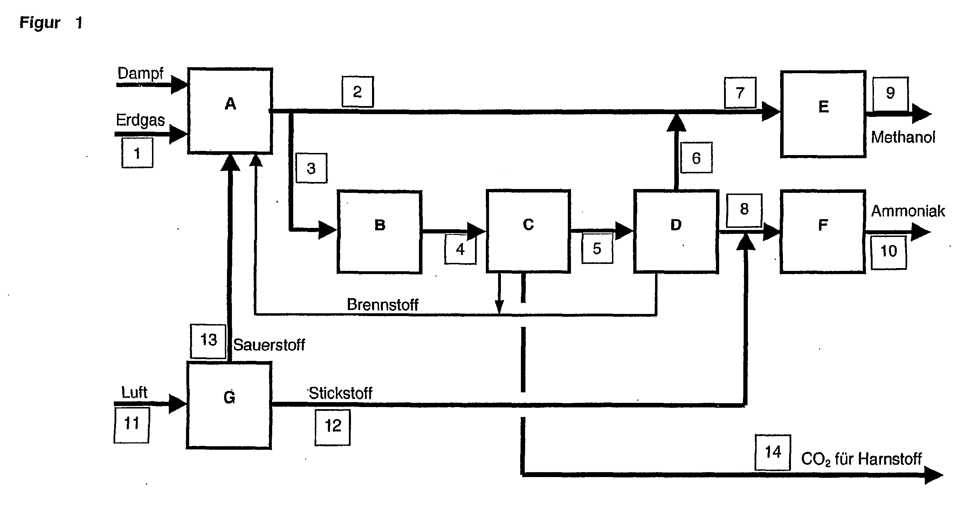

Die Erfindung wird im Folgenden beispielhaft anhand der Zeichnungen erläutert. Es zeigen
- Figur 1: eine Anordnung zur Koproduktion von Methanol und Ammoniak aus Erdgas, wie sie dem oben beschriebenen Verfahren ent- spricht,

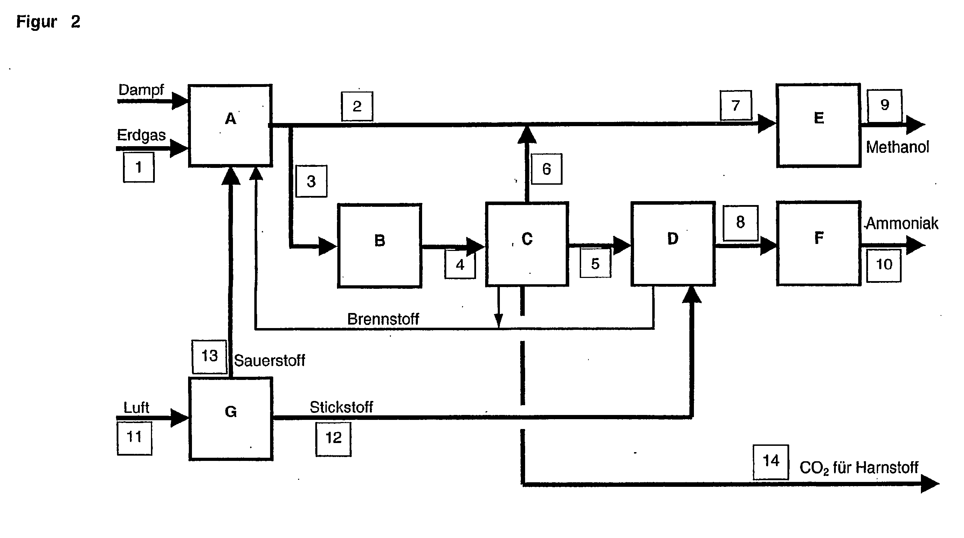

- Figur 2: eine Anordnung zur Koproduktion von Methanol und Ammoniak, wobei in der Reinigungseinheit D zusätzlich eine Wäsche mit flüs- sigem Stickstoff der Lufttrennungseinheit G (Strom 12) vorgese- hen ist. In diesem Fall wird der dem Methanol-Synthesegas zuge- leitete Wasserstoff (Strom 6) bereits dem Verdichter und Absorber C entnommen,

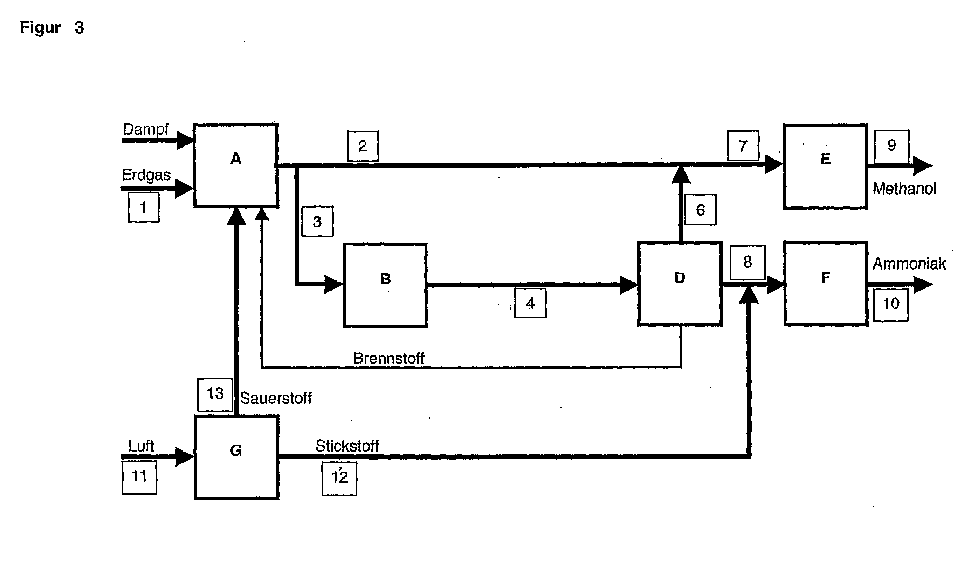

- Figur 3: eine Anordnung zur Koproduktion von Methanol und Ammoniak, wobei auf den Verdichter und Absorber C gänzlich verzichtet wird, und die Reinigungseinheit D die Druckwechselabsorption mit ein- schließt. In diesem Fall wird kein Kohlendioxid für die Harnstoff- synthese abgezweigt.

Im Reaktor A findet die Synthesegaserzeugung statt. Dabei können einige oder alle der folgenden Einzelschritte umfasst sein: Entschwefelung des Erdgases, Anfeuchtung bis hin zur Sättigung mit Wasserdampf, Vorheizung in einem Ofen, Präreformierung, katalytische partielle Oxidation mit Sauerstoff aus einer Lufttrennungseinheit sowie Gaskühlung in einem Abhitzekessel zur gesteigerten Dampferzeugung. Der dafür eingesetzte CPOX-Reaktor (catalytic partial oxidation) selbst ist ein herkömmlicher, zylindrischer Behälter mit konkaven, senkrecht angeordneten Begrenzungsflächen. Im oberen Ende des Behälters ist ein Brenner oder Mischer angebracht, durch den das Erdgas sich mit dem Wasserdampf vermischt. Dampf und Sauerstoff werden über separate Zuleitungen in den Behälter geführt. Der Sauerstoff kann beispielsweise der Lufttrennungseinheit G entnommen werden, in der aus Luft (Strom 11) durch Tieftemperaturdestillation die Komponenten Sauerstoff (Strom 13) und Stickstoff (Strom 12) abgetrennt werden. Der Brenner oder Mischer begünstigt ein gründliches Durchmischen der drei Gasströme in der oberen Hälfte des Behälters, wo der größte Teil der partiellen Oxidation sehr schnell stattfindet. Die heißen Gase treten dann über in einen Reformierungskatalysator, der in der unteren Hälfte des Behälters angeordnet ist, wo die Reformierung des Erdgases abgeschlossen wird. Die katalytische partielle Oxidation ist dabei typischerweise durch die folgenden fünf chemischen Reaktionen charakterisiert:

CH₄ + H₂O → CO + 3 H₂

CH₄ + 2 O₂ → CO₂ + 2 H₂O

2 CO + O₂ → 2 CO₂

2 H₂ + O₂ → 2 H₂O

CO₂ + H₂ = CO → H₂O.

Dem Erdgas (Strom 1) wird Dampf zugegeben, um ein Molverhältnis von nichtoxidiertem Kohlenstoff im Erdgas im Bereich zwischen 1.0 bis 3.0, normalerweise bei 1.7 für Hochdruckbetrieb, zu erzeugen. Im CPOX-Reaktor wird Sauerstoff zu dem nicht-oxidierten Kohlenstoff in einem Molverhältnis zwischen 0.45 und 0.7, üblicherweise bei 0.52, zugegeben. Die Sauerstoffmenge wird in der praktischen Anwendung präzise gesteuert, um eine Auslasstemperatur aus dem CPOX-Reaktor zwischen 900°C und 1.050°C zu erzeugen, normalerweise von 960°C. Die Reinheit des Sauerstoffs (Strom 13), der aus der Lufttrennungseinheit G bereitgestellt wird, liegt zwischen 90% und 99.5%, üblicherweise bei 99.5%. Der Katalysator, der in dem CPOX-Reaktor typischerweise benutzt wird, ist ein Nickeloxid-Katalysator, zum Beispiel vom Typ G-31 E, G-90LDP und G-90B von der Firma Süd-Chemie. Der Druck, bei dem die katalytische partielle Oxidationsreaktion ausgeführt wird, liegt zwischen 25 bar und 100 bar, normalerweise bei 40 bar.

Eine weitere Möglichkeit der Synthesegaserzeugung im Reaktor A besteht in der Kombination eines Dampfreformers mit dem CPOX-Reaktor. Dabei wird ein Teil des Erdgases zum Dampfreformer geleitet, in dem es bei Temperaturen zwischen 700°C und 950°C, vorzugsweise bei 780°C, in einem Molverhältnis von Dampf zu Kohlenstoff zwischen 1.5 und 3.0, vorzugsweise 2.0, und bei einem Druck zwischen 25 bar und 50 bar, vorzugsweise bei 40 bar, katalytisch zu Synthesegas gemäß folgenden Reaktionsgleichungen umgesetzt wird:

CH₄ + H₂O → CO + 3 H₂

CO₂ + H₂ → CO + H₂O.

Typischerweise wird hierfür ein Nickel/Aluminiumoxid-Katalysator, zum Beispiel vom Typ G-90LDP oder G-90B von der Firma Süd-Chemie, eingesetzt.

Anschließend wird das reformierte Erdgas mit dem Teil des Erdgasstroms, der in einem Bypass an dem Dampfreformer vorbeigeführt wird, gemischt und in den CPOX-Reaktor geleitet.

Alternativ kann auf eine Aufteilung des Erdgasstroms verzichtet werden, indem der gesamte Erdgasstrom zunächst durch den Dampfreformer und dann durch den CPOX-Reaktor gefahren wird.

Die Kombination eines Dampfreformers mit einem CPOX-Reaktor, insbesondere das Verfahren, bei dem das Erdgas mengenmäßig zwischen dem Dampfreformer und dem CPOX-Reaktor aufgeteilt wird, erhöht die Flexibilität des gesamten Verfahrens bezüglich der Gaszusammensetzung.

In dem Reaktor B wird das im Gasgemisch vorhandene Kohlenmonoxid in einem ein- oder zweistufigen Konversionskatalysator in Kohlendioxid umgewandelt, wobei zwischen den Stufen Kühlungen und hinter dem Reaktor B eine weitere Abkühlung des Stroms 4 und daraus resultierend eine Wärmegewinnung vorgesehen sind. Der Katalysator kann ein oder mehr Betten der bekannten Hochtemperatur-Konversionskatalysatoren und ebenfalls mindestens ein Bett der gleichfalls bekannten Niedertemperatur-Konversionskatalysatoren umfassen.

Im Verdichter und Absorber C wird das Rohgas gegebenenfalls bei erhöhtem Druck komprimiert, und das Kohlendioxid wird aus dem Gas entfernt. Die Entfernung von Kohlendioxid aus dem Rohsynthesegas wird mit einem Absorbens entweder durch eine physikalische Wäsche oder eine chemische Wäsche herbeigeführt. Im Fall einer physikalischen Wäsche ist das Absorbens typischerweise kaltes Methanol oder Glykolether. Im Fall einer chemischen Wäsche ist das Absorbens typischerweise ein Alkanolamin, ein Polyalkanolamin oder Kaliumcarbonat. Im Absorber wird das Kohlendioxid in dem Rohsynthesegas bis zu einem Gehalt von weniger als 50 ppmv, üblicherweise sogar von weniger als 10 ppmv, entfernt. Der Druck, bei dem das Kohlendioxid entfernt wird, liegt bei 30 bar bis 100 bar, normalerweise bei 80 bar. Der Verdichter und Absorber C schließt eine Einrichtung zur kontrollierten Druckminderung für das Absorbens zur Kohlendioxidwiedergewinnung ein, eine Einrichtung für die Regeneration des Absorbens durch Wärmezufuhr, ferner eine Einrichtung zur Aufrechterhaltung der richtigen Zusammensetzung des Absorbens und schließlich eine Einrichtung für die erneute Druckbeaufschlagung des Lösungsmittels bis auf die Höhe des Prozessdrucks. Das gesamte wieder gewonnene Kohlendioxid oder Teile davon können abgetrennt und beispielsweise für eine Harnstoffsynthese verwendet werden (Strom 14). Unverbrauchtes Kohlendioxid wird in die Atmosphäre abgeführt.

In der Reinigungseinheit D werden im Synthesegas verbliebene Verunreinigungen wie Methan, Spuren von Kohlenmonoxid und Argon aus dem Synthesegas ausgewaschen, beispielsweise mit flüssigem Stickstoff vermittels eines Molekularsiebs, allgemein bekannt als Druckwechselabsorption. Die Produkte der Reinigungseinheit D sind ein reiner Strom aus Ammoniak-Synthesegas, der im Fall einer Wäsche mit flüssigem Stickstoff das korrekte stöchiometrische Verhältnis aufweist, und bei Anwendung von Druckwechselabsorption ein reiner Strom von Wasserstoff. In beiden Fällen wird ein Strom von allen verbliebenen Verunreinigungen als Brenngas für die Öfen im Prozess verwendet. Der Druck, bei dem die Reinigungseinheit D betrieben wird, liegt zwischen 30 bar und 100 bar, normalerweise bei 75 bar bei einer Wäsche mit flüssigem Stickstoff und bei 30 bar bei Anwendung von Druckwechselabsorption. Wenn die Reinigungseinheit D eine Wäsche mit flüssigem Stickstoff umfasst, dann wird ein Molekularsieb zur Entfernung von Spuren von Kohlendioxid und von einem Absorbens in dem kohlendioxidfreien Synthesegas vorgeschaltet.

In der Einheit E wird das Methanol-Synthesegas (Strom 7) durch einen Katalysator in Methanol gewandelt und das Methanol durch Destillation auf die erforderliche Reinheit raffiniert (Strom 9). Der Druck, bei dem die Methanolsynthese stattfindet, liegt zwischen 60 bar und 120 bar, normalerweise bei 70 bar. Die Methanoldestillation findet ungefähr bei 15 bar bis Atmosphärendruck statt.

In der Einheit F wird das Ammoniak-Synthesegas (Strom 8) komprimiert und durch einen Katalysator in Ammoniak gewandelt (Strom 10). Das Ammoniak wird vom wieder gewonnenen Synthesegas durch partielle Kondensation bei Niedrigtemperaturen abgetrennt, wobei als Kühlmittel flüssiges Ammoniak benutzt wird. Der Druck, bei dem die Ammoniaksynthese stattfindet, liegt zwischen 120 bar und 250 bar, normalerweise bei 200 bar.

In der Lufttrennungseinheit G werden aus der Luft die Komponenten Sauerstoff (Strom 13) und Stickstoff (Strom 12) durch Tieftemperaturdestillation voneinander getrennt. Der Prozess der Luftaufspaltung in ihre Bestandteile ist wohlbekannt. Die Produkte aus der Lufttrennungseinheit G umfassen einen Sauerstoffstrom mit einer Reinheit zwischen 90% und 99.5%, normalerweise bei 99.5%, einen Strom aus Stickstoff mit einer Reinheit von mehr als 99.995% und einen Strom aus Abfallgas mit den Abfallprodukten Sauerstoff und Stickstoff, die normalerweise in die Atmosphäre abgelassen werden. Die Lufttrennungseinheit G kann auch, falls gewünscht, einen oder mehr als einen Strom von den seltenen Gasen wir Argon, Helium und Neon produzieren.

## Patentansprüche

1. Verfahren zur Koproduktion von Methanol und Ammoniak aus Erdgas, **gekennzeichnet durch** die folgenden Schritte:
1.1 In einem Reaktor A werden Erdgas (Strom 1), Dampf und **einer Lufttrennungseinheit G entnommener** Sauerstoff (Strom 13) miteinander vermischt, wobei das Erdgas partiell oxidiert wird und mit Hilfe von Katalysatoren weiter reformiert wird,
1.2 das dem Reaktor A entnommene Gasgemisch wird in einen Strom (Strom 2) für die Methanolsynthese in einer Einheit E und einen weiteren Strom (Strom 3) für die Wasserstoffproduktion aufgeteilt,
1.3 das im Strom (Strom 3) für die Wasserstoffproduktion vorhandene Kohlenmonoxid wird in einem Reaktor B mit Hilfe von Katalysatoren und Zwischenkühlungsstufen in Kohlendioxid gewandelt,
1.4 in einer Reinigungseinheit D werden verbliebene Verunreinigungen wie Methan, Spuren von Kohlenmonoxid und Argon **mit aus einer Lufttrennungseinheit G entnommenem flüssigen Stickstoff (Strom 12)** ausgewaschen, und Wasserstoff (Ströme 6, 8) wird der Methanolsynthese in der Einheit E und der Ammoniaksynthese in einer Einheit F zugeführt, **wobei verbliebene Verunreinigungen als Brennstoff für die Öfen des Prozesses verwendet werden,**
1.5 in der Einheit E wird Methanol-Synthesegas (Strom 7) mit Hilfe eines Katalysators in Methanol (Strom 9) umgewandelt, und das Methanol wird durch Destillation auf die erforderliche Reinheit gebracht,
1.6 in der Einheit F wird Ammoniak-Synthesegas (Strom 8) komprimiert und mit Hilfe eines Katalysators in Ammoniak (Strom 10) umgewandelt, und das Ammoniak wird vom wieder gewonnenen Synthesegas **durch** partielle Kondensation getrennt.

2. Verfahren nach Anspruch 1, **dadurch gekennzeichnet, dass** im Schritt 1.1 ein Teil des Erdgases zuerst durch einen Dampfreformer geleitet, anschießend mit dem übrigen Erdgas gemischt und in einen CPOX-Reaktor geleitet wird.

3. Verfahren nach Anspruch 1 oder 2, **dadurch gekennzeichnet, dass** in einem Verdichter und Absorber C das aus dem Reaktor B tretende Gasgemisch (Strom 4) komprimiert wird, das Kohlendioxid ausgewaschen wird und das Gasgemisch (Strom 5) der Reinigungseinheit D zugeleitet wird.

4. Verfahren nach Anspruch 3, **dadurch gekennzeichnet, dass** im Verdichter und Absorber C eine physikalische Wäsche mit Hilfe eines geeigneten Absorbens, insbesondere mit kaltem Methanol oder Glykolether, durchgeführt wird.

5. Verfahren nach Anspruch 3, **dadurch gekennzeichnet, dass** im Verdichter und Absorber C eine chemische Wäsche mit einem geeigneten Absorbens, insbesondere einem Alkanolamin, ein Polyalkanolamin oder Kaliumcarbonat, durchgeführt wird.

6. Verfahren nach einem der Ansprüche 3 bis 5, **dadurch gekennzeichnet, dass** das im Verdichter und Absorber C gewonnene Kohlendioxid (Strom 14) zur Harnstoffherstellung verwendet wird.

7. Verfahren nach einem der Ansprüche 3 bis 6, **dadurch gekennzeichnet, dass** der dem Methanol-Synthesegas zugeleitete Wasserstoff (Strom 6) dem Verdichter und Absorber C entnommen wird.

## Claims

1. A method for coproducing methanol and ammonia from natural gas **characterized by** the following steps:
1.1 In a reactor A, natural gas (flow 1), steam and oxygen withdrawn from an air separation unit G (flow 13) are mixed with one another, the natural gas being partially oxidized and further reformed with the aid of catalysts;
1.2 the gas mixture withdrawn from reactor A is split into a flow (flow 2) for methanol synthesis in a unit E and a further flow (flow 3) for hydrogen production;
1.3 the carbon monoxide present in the flow (flow 3) for hydrogen production is converted into carbon dioxide with the aid of catalysts and intermediate cooling stages in a reactor B;
1.4 in a purifying unit D, remaining impurities such as methane, traces of carbon monoxide and argon are removed by scrubbing with liquid nitrogen (flow 12) withdrawn from an air separation unit G, and hydrogen (flows 6, 8) is fed to the methanol synthesis in unit E and to the ammonia synthesis in unit F, remaining impurities being used as fuel for the furnaces of the process;
1.5 in unit E, methanol synthesis gas (flow 7) is converted into methanol (flow 9) with the aid of a catalyst, and the methanol is brought to the required purity level by distillation;
1.6 in unit F, ammonia synthesis gas (flow 8) is compressed and converted into ammonia (flow 10) with the aid of a catalyst, and the ammonia is separated from the recovered synthesis gas by partial condensation.

2. A method according to claim 1, **characterized in that** in step 1.1, a portion of the natural gas is first passed through a steam reformer, subsequently mixed with the remaining natural gas, and introduced into a CPOX reactor.

3. A method according to claim 1 or claim 2, **characterized in that** in a compressor and absorber C, the gas mixture flowing out of reactor B (flow 4) is compressed, the carbon dioxide is removed by scrubbing, and the gas mixture (flow 5) is fed to purifying unit D.

4. A method according to claim 3, **characterized in that** in compressor and absorber C, a physical scrubbing with the aid of a suitable absorbent, in particular with cold methanol or glycol ether, is performed.

5. A method according to claim 3, **characterized in that** in compressor and absorber C, a chemical scrubbing with a suitable absorbent, in particular an alkanolamine, a polyalkanolamine or potassium carbonate, is performed.

6. A method according to any one of claims 3 to 5, **characterized in that** the carbon dioxide obtained in compressor and absorber C (flow 14) is used for producing urea.

7. A method according to any one of claims 3 to 6, **characterized in that** the hydrogen fed to the methanol synthesis gas (flow 6) is withdrawn from compressor and absorber C.

## Revendications

1. Procédé de production simultanée de méthanol et d'ammoniaque à partir de gaz naturel, **caractérisé par** les étapes suivantes :
1.1 Dans un réacteur A, on mélange du gaz naturel (flux 1), de la vapeur et de l'oxygène issu d'une unité de séparation d'air G (flux 13), ledit gaz naturel étant ainsi partiellement oxydé pour ensuite poursuivre son reformage à l'aide de catalyseurs,
1.2 le mélange gazeux issu du réacteur A est partagé en un flux (flux 2) destiné à la synthèse de méthanol dans une unité E et un autre flux (flux 3) destiné à la production d'hydrogène,
1.3 le monoxyde de carbone contenu dans le flux (flux 3) destiné à la production d'hydrogène est transformé en dioxyde de carbone à l'aide de catalyseurs et d'étages de refroidissement intermédiaire,
1.4 dans une unité de purification D, les impuretés résiduelles telles que le méthane, les traces de monoxyde de carbone et d'argon sont enlevées par lavage avec de l'azote liquide (flux 12) issu d'une unité de séparation d'air G, et de l'hydrogène (flux 6, 8) est acheminé vers la synthèse de méthanol dans l'unité E et vers la synthèse d'ammoniaque dans l'unité F, les impuretés résiduelles étant utilisées comme combustible dans les fours du processus,
1.5 dans l'unité le gaz destiné à la synthèse de méthanol (flux 7) est transformé en méthanol (flux 9) à l'aide d'un catalyseur et le méthanol est purifié par distillation jusqu'à ce qu'il corresponde aux exigences de pureté,
1.6 dans l'unité F, le gaz destiné à la synthèse d'ammoniaque (flux 8) est comprimé et transformé en ammoniaque (flux 10) à l'aide d'un catalyseur puis l'ammoniaque est séparé par condensation partielle du gaz de synthèse récupéré.

2. Procédé selon la revendication 1, **caractérisé en ce que,** dans l'étape 1.1, on fait d'abord passer une partie du gaz naturel par un dispositif de reformage à la vapeur pour ensuite le mélanger au reste du gaz naturel et introduire ce mélange dans un réacteur de type CPOX.

3. Procédé selon la revendication 1 ou 2, **caractérisé en ce que**, dans un dispositif de compression et d'absorption C, le mélange gazeux issu du réacteur B (flux 4) est comprimé et subit une élimination du dioxyde de carbone par lavage, le mélange gazeux (flux 5) étant acheminé vers l'unité de purification D.

4. Procédé selon la revendication 3, **caractérisé en ce qu'**on réalise, dans le dispositif de compression et d'absorption C, un lavage physique à l'aide d'un absorbant approprié, s'agissant plus particulièrement de méthanol ou d'éther de glycol froids.

5. Procédé selon la revendication 3, **caractérisé en ce qu'**on réalise, dans le dispositif de compression et d'absorption C, un lavage chimique à l'aide d'un absorbant approprié, s'agissant plus particulièrement d'une alcanolamine, d'une polyalcanolamine ou de carbonate de potassium.

6. Procédé selon l'une des revendications 3 à 5, **caractérisé en ce que** le dioxyde de carbone (flux 14) obtenu dans le dispositif de compression et d'absorption C est utilisé pour préparer de l'urée.

7. Procédé selon l'une des revendications 3 à 6, **caractérisé en ce que** l'hydrogène (flux 6), qui est acheminé vers le gaz destiné à la synthèse de méthanol, est issu du dispositif de compression et d'absorption C.
